# EUROPEAN PATENT SPECIFICATION

(11) **EP 3 924 466 B1**
(45) Date of publication and mention of the grant of the patent: **14.05.2025**
(21) Application number: 20703264.0
(22) Date of filing: 12.02.2020
(51) Int. Cl.: C12N 5/073

(54) **COMPOSITION FOR IMPROVING THE CULTURE AND IMPLANTATION OF MAMMALIAN EMBRYOS, PREPARATION METHOD AND USE THEREOF**
ZUSAMMENSETZUNG ZUR VERBESSERUNG DER ZÜCHTUNG UND DER IMPLANTATION VON SÄUGETIEREMBRYONEN, HERSTELLUNGSVERFAHREN UND VERWENDUNG DAVON
COMPOSITION POUR AMÉLIORER LA CULTURE ET L'IMPLANTATION D'EMBRYONS DE MAMMIFÈRE, SON PROCÉDÉ DE PRÉPARATION ET SON UTILISATION

(30) Priority: 15.02.2019 EP 19382106
(43) Date of publication of application: 22.12.2021
(73) Proprietor: Grifols Worldwide Operations Limited, Dublin 22 (IE); Fundació Institut d'Investigació Biomèdica de Bellvitge (IDIBELL), 08908 L'Hospitalet de Llobregat (Barcelona) (ES)
(72) Inventor: COSTA RIEROLA, Montserrat, 08150 Parets Del Valles (Barcelona) (ES); ORTIZ FERNANDEZ, Ana Maria, 08150 Parets Del Valles (Barcelona) (ES); OJOSNEGROS MARTOS, Samuel, 08017 Barcelona (ES); SERIOLA PETIT, Anna, 08028 Barcelona (ES)
(74) Representative: Durán-Corretjer, S.L.P.
(86) International application number: PCT/EP2020/053548
(87) International publication number: WO 2020/165218

(56) References cited:
- POOL T B ET AL: "High continuing pregnancy rates after in vitro fertilization-embryo transfer using medium supplemented with a plasma protein fraction containing alpha- and beta-globulins", vol. 61, no. 4, 31 March 1994 (1994-03-31), pages 714 - 719, XP009512604, ISSN: 0015-0282, Retrieved from the Internet <URL:https://www.fertstert.org/article/S0015-0282(16)56651-5/pdf> DOI: 10.1016/S0015-0282(16)56651-5
- MORBECK DEAN E ET AL: "Composition of protein supplements used for human embryo culture", JOURNAL OF ASSISTED REPRODUCTION AND GENETICS, PLENUM PUBLISHING, US, vol. 31, no. 12, 27 September 2014 (2014-09-27), pages 1703 - 1711, XP035383132, ISSN: 1058-0468, [retrieved on 20140927], DOI: 10.1007/S10815-014-0349-2
- DALIT BEN-YOSEF ET AL: "Prospective Randomized Comparison of Two Embryo Culture Systems: P1 Medium by Irvine Scientific and the Cook IVF Medium", JOURNAL OF ASSISTED REPRODUCTION AND GENETICS., vol. 21, no. 8, 1 August 2004 (2004-08-01), US, pages 291 - 295, XP055580791, ISSN: 1058-0468, DOI: 10.1023/B:JARG.0000043702.35570.56
- IRINA A. TARASOVA ET AL: "Depletion of human serum albumin in embryo culture media for in vitro fertilization using monolithic columns with immobilized antibodies : Proteomics and 2-DE", ELECTROPHORESIS, vol. 37, no. 17-18, 1 September 2016 (2016-09-01), pages 2322 - 2327, XP055580793, ISSN: 0173-0835, DOI: 10.1002/elps.201500489
- SWAIN JASON E ET AL: "Optimizing the culture environment and embryo manipulation to help maintain embryo developmental potential", FERTILITY AND STERILITY, ELSEVIER SCIENCE INC, NEW YORK, NY, USA, vol. 105, no. 3, 3 February 2016 (2016-02-03), pages 571 - 587, XP029449941, ISSN: 0015-0282, DOI: 10.1016/J.FERTNSTERT.2016.01.035
- MCCANN KARL B ET AL: "Use of mep HyperCel for polishing of human serum albumin", JOURNAL OF CHROMATOGRAPHY B: BIOMEDICAL SCIENCES & APPLICATIONS, ELSEVIER, AMSTERDAM, NL, vol. 969, 28 August 2014 (2014-08-28), pages 241 - 248, XP029057109, ISSN: 1570-0232, DOI: 10.1016/J.JCHROMB.2014.08.029
- KAMRAN MOUSAVI HOSSEINI ET AL: "Implementation of Plasma Fractionation in Biological Medicines Production", IRANIAN JOURNAL OF BIOTECHNOLOGY, vol. 14, no. 4, 28 December 2016 (2016-12-28), IR, pages 213 - 220, XP055580805, ISSN: 1728-3043, DOI: 10.15171/ijb.1401

## Description

The present invention relates to the field of assisted reproductive technology. In particular, it relates to a composition for improving the culture and implantation of mammalian embryos comprising one or more of the fractions of human plasma fractionation according to the Cohn method enriched with human serum albumin (HSA). In addition, the present invention relates to the preparation method and use of said composition in the culture of pre-implantation mammalian embryos.

Today, assisted reproductive technology is available throughout most of the developed countries and the practice is largely different from that used during the early days. Refinements in laboratory technology and clinical practice have allowed *in vitro* fertilization (IVF) to evolve into a medical procedure that is efficient, safe, readily accessible, and relatively affordable.

IVF is the joining of a woman's egg and a man's sperm in a laboratory dish. *In vitro* means outside the body. Fertilization means the sperm has attached to and entered the egg. The fertilized egg is an embryo. Embryo development involves a series of cell divisions. Said embryo is cultured and is checked to make sure it is developing properly.

The culture of embryos is a key and critical step for selection of viable and genetically normal embryos. Therefore, an improved culture of mammalian embryos should enable the transfer of embryos and resulting in an increased implantation rate. Trying to improve the culture media is arguably the most common approach to promote embryo development *in vitro.*

Several factors affect the success of IVF. Proper embryo culture conditions and medium formulation, in particular, are two indispensable requirements (Mauri et al., Clinical assisted reproduction: a prospective, randomized comparison of two commercial media for ICSI and embryo culture, J Assist Reprod Genet, 2001;18(7):378-381; Summers and Biggers, Chemically defined media and the culture of mammalian preimplantation embryos: historical perspective and current issues, Hum Reprod Update, 2003;9(6):557-82; Hentemann and Bertheussen, New media for culture to blastocyst, Fertil Steril, 2009;91(3):878-83; Paternot et al., Early embryo development in a sequential versus single medium: a randomized study, Reprod Biol Endocrinol, 2010;8:83). Thus, to obtain mammalian embryos that successfully implant into the uterus, an improved embryo culture medium is required that mimics the phisiological conditions. In general, two possible formulations have been considered in the prior art for culture media: one is based on a sequential embryo culture medium and the other is based on a single culture medium.

The sequential embryo culture medium involves switching the embryo sequentially to two different media, the first one can drive the embryo from the one-cell stage up to the 8-cell stage, the second one from 8-cell to the blastocyst stage. The sequential medium tries to mimic the variable environmental conditions meet by the embryo first in the Fallopian tubes and later in the uterus.

The single culture medium provides the embryo with all the ingredients required at the different stages of develoment, and therefore does not require to switch the embryo to different plates. Currently, the continuos medium is implemented in most assisted reproduction centers.

When the embryos reach the blastocyst stage, their metabolic requirements are different from the pre-implantation embryos ones. To satisfy these metabolic demands and to ensure a proper embryo development, the culture media must have a higher protein concentration.

Back in the initial years of IVF, human serums were used for embryo culture, although they were discarded due to their low safety against contamination from possible infections from donors (Blake et al., Protein supplementation of human IVF, J Assist Reprod Genet, 2002;19(3):137-43; Leveille et al., Effects of human sera and human serum albumin on mouse embryo culture, Genet, 1992;9(1):45-52). Progressively, both human plasma-derived and recombinant albumin have been used as a protein supplement. Supplementing with purified albumin alone, however, does not allow the development of the embryo beyond the blastocyst stage. Nowadays, there are a number of culture media that allow the transition of pre-implantation embryos (blastocysts) to their post-implantation *in vitro* development. These culture media are however experimental, or include components such as hormones, xenobiotic serums or human umbilical cord serum which do not ensure GMP quality and, therefore, cannot be approved for clinical use (Morris et al., Dynamics of previous-posterior axis formation in the developing mouse embryo, Nat Commun, 2012;3:673, Ajduk and Zernicka-Goetz, Advances in embryo selection methods, F1000 Biol Rep, 2012;4:11; Shahbazi et al., Self-organization of the human embryo in the absence of maternal tissues, Nat Cell Biol, 2016;18(6):700-708; Bedzhov et al., In vitro culture of mouse blastocysts beyond the implantation stages, Nat Protocol, 2014;9(12):2732-9; Deglincerti et al., Self-organization of the in vitro-linked human embryo, Nature, 2016;533(7602):251-4).

In the last few years, assisted reproductive technology has undergone progressive optimization. However the pregnancy rate per embryo transferred is still low (~30 %). The cornerstone to increase the efficiency of IVF is the embryo implantation (Zhang et al., Physiological and molecular determinants of embryo implantation, Mol Aspects Med, 2013;34(5):939-80). Currently, different methods, both experimental and clinical, try to improve the efficiency of embryo implantation, with very moderate or low success.

Successful implantation requires synchronization between the acquisition of implantation competency by the blastocyst and a receptive state in the uterine endometrium (Dey et al., Molecular cues to implantation, Endocr Rev, 2004;25(3)341-373; Tranguch et al., Molecular complexity in establishing uterine receptivity and implantation, Cell Mol Life Sci, 2005;62(17)1964-1973; Wang and Dey, Roadmap to embryo implantation: clues from mouse models, Nat Rev Genet, 2006;7(3)185-199).

The use of time-lapse incubators which incorporate a built-in microscope allows the simultaneous and uninterrupted observation of embryos without the need to remove them from the incubator. To accommodate this set-up, a single culture medium was developed, which allows the culture of embryos from the 1-cell stage to blastocyst. This embryo culture medium replaces the sequential culture medium, which requires one or two changes of medium during the process of embryo culture. Although some studies show that the single uninterrupted culture can improve the culture efficiency during the early stages, the benefit of this medium in later stages, from morula to blastocyst, has no statistical support (Sciorio et al., Comparison of the development of human embryos cultured in either an EmbryoScope or benchtop incubator, J Assist Reprod Genet, 2018;35(3):515-522). Retrospective studies suggest that the single-medium culture can globally improve the efficiency of *in vitro* culture, also improving the implantation rate, but does not have a real, significant impact on pregnancy rates (Alhelou et al., Embryo culture conditions are significantly improved during uninterrupted incubation: a randomized controlled trial, Reprod Biol, 2018;18(1):40-45). Time-lapse follow-up of embryo development comprises an undisturbed culture and the application of the visual information to support embryo evaluation. The selection of embryos for transfer in the time-lapse culture is based on image monitoring combined with the image analysis of a morphokinetic algorithm for embryo selection. However, a thorough statistical assesment of these algortims indicates that (i) not all algorithms are equally predictive; external parameters, such as (ii) the initial morphology of the embryo and (iii) the age of the mother have a greater impact in the determination.

Furthermore, preimplantation Genetic Diagnosis (PGD) involves biopsying embryos in culture to get few cells to perform a genetic analysis. This is an invasive method that provides information on whether an embryo is affected by a monogenic disease and/or chromosomal impairments (aneuploidies). PGD however does not offer predictive information on the ability of the embryo to implant (Cimadomo et al., The impact of biopsy on human embryo developmental potential during preimplantation genetic diagnosis, Biomed Res Int, 2016;2016:7193075). Moreover, most pre-implantation embryos are genetically mosaic, meaning that different cells have different genetic background. This is particularly true for biopsies performed on early embryos (i.e. 8-cell stage). Retroactive studies show that the use of PGD does not significantly improves the embryo implantation rate (Geraedts and Sermon, Preimplantation genetic screening 2.0: the theory. Mol Hum Reprod, 2016;22(8):839-44; Sermon et al., The why, the how and the when of PGS 2.0: current practices and expert opinions of fertility specialists, molecular biologists, and embryologists. Mol Hum Reprod, 2016;22(8):845-57).

In summary, the embryo culture from zygote stage (one cell) to the blastocyst stage is relatively optimized. However, once the embryos have been transferred to the patient, implantation success is still relatively low. For this reason, it is necessary to develop new culture components that either improve the ability of embryos to implant once transferred to the uterus, or generate robust embryos at the stage of transfer.

The inventors have carried out intensive and extensive research aimed at using different culture media with human plasma fractions and they have found, surprisingly, that by using a composition comprising one or more of the fractions of the Cohn method wherein in said composition HSA is between 90 % and 96 % of the total proteins in the composition, alfa and beta globulins are between 3.5 % and 9.99 % of the total proteins in the composition, and gamma globulin is between 0.01 % and 0.5 % of the total proteins in the composition, it is possible to improve *in vitro* embryo culture and to obtain better results in their implantation in comparison with embryos cultured under the conditions of the prior art. For example, these better results are shown by a higher percentage of embryos with complete hatching success.

In the present invention, the term "Cohn method" refers to the method of separating blood plasma proteins developed by Edwin J. Cohn (Cohn et al., Preparation and properties of serum plasma proteins, IV. A system for the separation into fractions of the protein and lipoprotein components of biological tissues and fluids, J. Am. Chem. Soc, 1946;68:459-475) or to any of its variants.

One further advantage of the composition of the present invention is that it can be obtained from human plasma.

Therefore, the present invention discloses a composition for the culture of mammalian embryos the use of which results in a new therapeutic tool for assisted reproduction technology specialists. The effectiveness of the use of the composition obtained by the method disclosed in the present invention is based on the use of one or more of the fractions of the Cohn method enriched with HSA that, together, induce the adhesion of embryos to the culture plate improving the implantation of said mammalian embryos.

In a first aspect, the present invention discloses a composition for improving the implantation of mammalian embryos comprising the supernatant of Fraction II+III and the Fraction V of the Cohn method wherein in said composition human serum albumin (HSA) is between 90 % and 96 % of the total proteins in the composition, alfa and beta globulins are between 3.5 % and 9.99 % of the total proteins in the composition, and gamma globulin is between 0.01 % and 0.5 % of the total proteins in the composition wherein said HSA is at a final concentration of 50 mg/ml to 250 mg/ml.

In a particular embodiment, the supernatant of Fraction II+III of the Cohn method can be further processed. Once the supernatant of Fraction II+III is obtained, it is subjected to different stages until the final product is obtained. These stages usually include: dilution, clarification, diafiltration, nanofiltration, concentration by ultrafiltration, sterilisation, filling into vials and final lyophilisation of said vials, prior to submission of said vials to gamma irradiation, or a combination thereof.

In another particular embodiment, the Fraction V of the Cohn method can be further processed. Once Fraction V is obtained, the latter is resuspended in a solution and is subjected to different stages until the final product is obtained. These stages usually include: diafiltration, heat treatment, sterilisation, filling into vials and final pasteurization of said vials, prior to submission of said vials to quarantine, in general during a period of not less than 14 days at 30-32 °C, with the objective of ensuring the sterility of the final product, or a combination thereof.

In another particular embodiment, the supernatant of Fraction II+III and the Fraction V, as such or as further processed production intermediates or final products, are submitted to one or more production steps with capacity to eliminate pathogens.

Preferably, said HSA is present in the composition at a final concentration of 100 mg/ml.

Moreover, the present invention discloses a method for the preparation of a embryo culture medium comprising the composition of the present invention comprising the following steps:
a) obtaining the composition of the present invention with the method disclosed above;
b) adding 10-50 % (v/v) of said composition to a mammalian embryo culture.

Preferably, the composition of the present invention is present in the embryo culture medium at 20 %.

Finally, the present invention discloses the use of a composition as described above for the culture of pre-implantation mammalian embryos. The implantation of the embryo is not part of the method of the invention.

Hereinafter, the present invention is described with reference to examples, which, however, are not intended to limit the present invention.

The present invention will be described below in reference to the figures, in which:
Figure 1 is a graphic of the results showing the improved performance of composition III (composition of the present invention) for the culture of mammalian embryos at the bastocyst stage compared to composition I and composition II. The improvement of said culture was measured as the percentage of embryos that progress sequentially from "bleb" to "half hatcihng", until they reach the complete "hatching". "Arrested" refers to those embryos blocked at blastocyst, which did not develope further.
Figure 2 is a graphic of the results of the adhesion of the hatched embryos to the culture plate surface in the culture using composition I and composition III.
Figure 3 is an illustration of confocal microscopy images of the implantation of embryos in collagen hydrogels using using composition I and composition III.

Hereinafter, the present invention is described with reference to examples, which however are not intended to limit the present invention.

### EXAMPLES

EXAMPLE 1: Preparation and characterization of the composition of the present invention and comparative compositions.

Composition I (table I) differs from composition III in that it has a higher amount of albumin and a lower amount of alpha-1 globulin. On the other hand, composition II differs from composition III in that it has a lower amount of albumin and a lower amount of alpha-1 globulin.

The composition of the majority components of said compositions is shown in the table below:

**Table I: Composition of the majority components of compositions I, II and III (composition of the present invention).**

| | Composition I n=3 (x±SD) | Composition II n=4 (x±SD) | Composition III (present invention) n=3 (x±SD) |
|---|---|---|---|
| Seroalbumin (g/L) | 198.1±0.4 | 22.7±1.8 | 107.6±1.5 |
| Alpha-1 globulin (g/L) | 2.3±0.2 | 1.3±0.2 | 6.4±0.4 |
| Alpha-2 globulin (g/L) | Not detected | 2.7±0.7 | 2.2±0.2 |
| Beta globulin (g/L) | Not detected | 2.2±0.4 | 1.6±0.3 |
| Gamma globulin (g/L) | Not detected | 0.5±0.1 | 0.5±0.1 |
| Seroalbumin (%) | 98.9±0.1 | 77.1±2.2 | 91.0±0.5 |
| Alpha-1 globulin (%) | 1.1±0.1 | 4.5±0.3 | 5.4±0.4 |
| Alpha-2 globulin (%) | Not detected | 9.0±1.4 | 1.8±0.2 |
| Beta globulin (%) | Not detected | 7.5±0.7 | 1.3±0.2 |
| Gamma globulin (%) | Not detected | 1.6±0.3 | 0.4±0.1 |
| Total protein (g/L) | 200.3±0.3 | 29.5±3.2 | 118.3±1.1 |

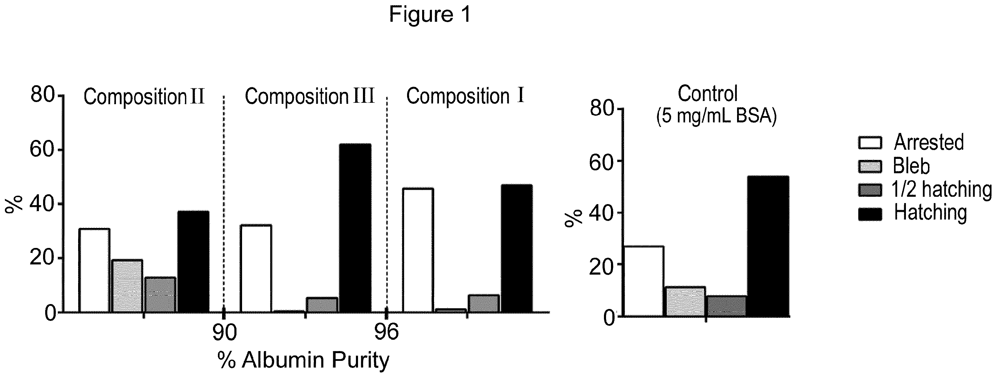

EXAMPLE 2: Evaluation of the development of mouse embryos in the presence of the composition of the present invention and comparative compositions (shown in Figure 1).

For determining the efficiency of the embryo culture, different protein formulations were prepared using as a reference the final concentration of HSA. KSOMAA (Millipore; USA) was used as a base medium with bovine serum albumin (BSA, 5 mg/ml) for bringing the mouse embryo culture E0.5 to the blastocyst stage (E3.5). Upon arrival at the blastocyst stage some embryos remained as a control in KSOMAA (BSA 5 mg/ml) and the rest of the embryos were transferred to a new KSOMAA medium without BSA and supplemented with:
- Group I: composition I based on a fraction of the human plasma fractionation using the Cohn method comprising more than 96 % of HSA, less than 3.5 % of alfa and beta globulins and less than 0.5 % of gamma globulins. The fraction should be added at a final concentration of 22.5 mg/ml of HSA.
- Group II: composition II based on a fraction of the human plasma fractionation using the Cohn method comprising between 70 % and 90 % of HSA, between 9.5 % and 28 % of alfa and beta globulins and between 0.5 % and 2 % of gamma globulins. The fraction should be added at a final concentration of 5 mg/ml of HSA.
- Group III (composition III, which is the composition of the present invention): a fraction of the human plasma fractionation using the Cohn method comprising between 90 % and 96 % of HSA, between 3.5 % and 9.99 % of alfa and beta globulins and between 0.01 % and 0.5 % of gamma globulins. The fraction should be added at a final concentration of 22.5 mg/ml of HSA.

Embryos at the blastocyst stage (E3.5) were left in culture in the incubator until day E6.5 to allow complete hatching. Their morphology was recorded daily through photographs. At the end of the incubation period (E6.5), the stage of development of the embryos in culture was evaluated.

As shown in figure 1, the highest percentage of embryos with complete hatching success were those cultured in the presence of the composition of Group III which is the composition of the present invention. Specifically, 62 % of embryos in the presence of the composition of Group III managed to hatch, while in the presence of the composition of Group I 46.8 % did. Embryos cultured in the presence of BSA (5 mg/ml) or in the presence of the composition of Group II hatched with an efficiency of 53.9 % and 37.2 %, respectively. The distribution of embryos at different stages of development shows that embryos grown in the presence of the composition of Group III have two preferential stages, either they are still in blastocyst, or most progress directly to complete hatching. Under these conditions, few embryos were at intermediate steps, for example they were not detected at bleb stage. These results suggest that the composition of the present invention allows progress in the development of viable embryos more efficiently than under the other conditions.

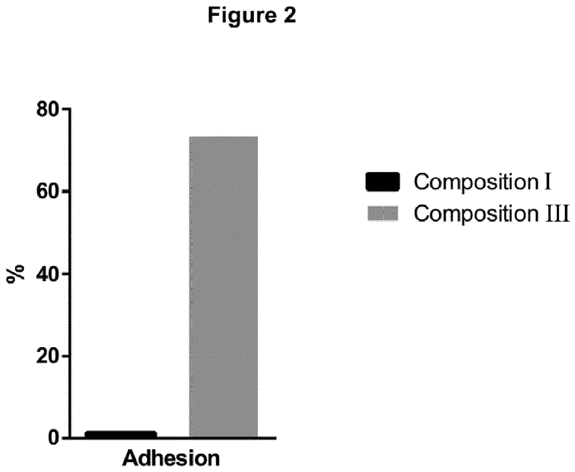

EXAMPLE 3: Evaluation of the adhesion of the embryos hatched in the culture (shown in Figure 2).

In this example, hatched mouse embryos are able to adhere to the culture plate and proliferate if they are grown under optimal conditions. The adhesion involves an outgrowth of the embryo trophoblast attaching to the glass or plastic surface, which cannot be re-attached by flushing with a pippete. In figure 2, measurements of the adhesion capacity of blastocysts cultured from stage E3.5 to E7.5 in the presence of the composition of Group I were compared to embryos cultured with the composition of Group III. The results showed a surprising increase in the adhesion of hatched blastocysts when they were cultured in the presence of the composition of Group III. Specifically, 73.3 % of the embryos cultured in the composition of Group III adhered to the plate, while, at most, 1.3 % of embryos were able to adhere in the presence of the composition of Group I. Under the other two conditions only a marginal or zero percentage managed to adhere to the plate.

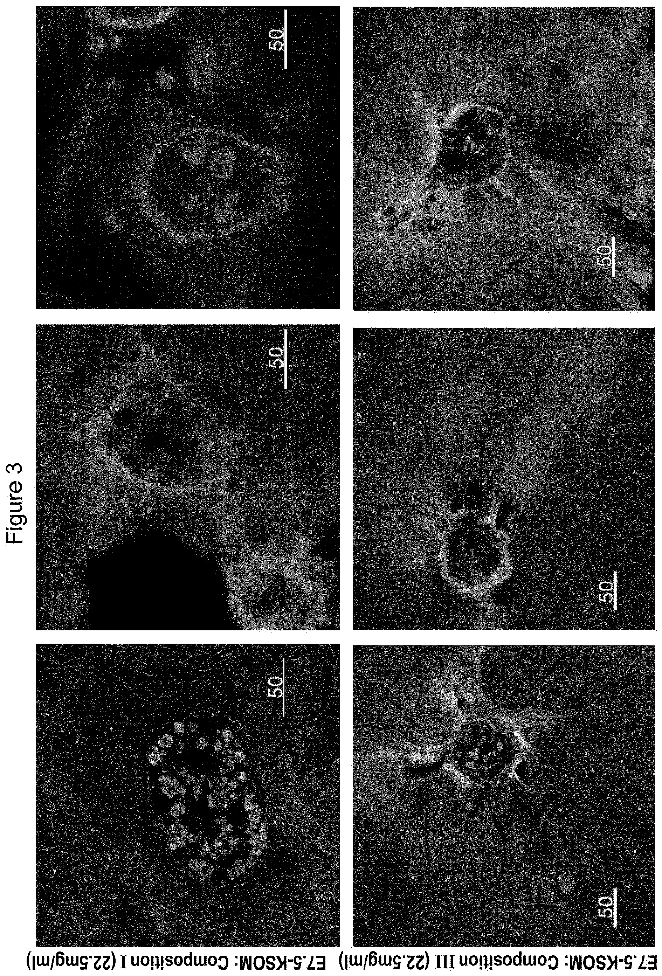

EXAMPLE 4: Implantation of embryos in collagen hydrogels (shown in Figure 3).

In preliminary studies, mouse embryos were embedded at the blastocyst stage within collagen hydrogels to allow their implantation (US 20150305774 A1). Confocal microscopy allows the observation of the transgenic fluorescent embryo in three dimensions within the implantation matrices. The collagen fibers can be observed by the reflection technique, and appear as a destructured mesh of white fibers, distributed in a relatively homogeneous way. As shown in figure 3, embryos cultured in the presence of the composition of Group III induce in certain areas in contact with the surface of the embryo, a type of dramatic remodeling in the fibers. This type of matrix remodeling suggests that the embryo is implanted in the matrix by strongly adhering and exerting force on it as documented during *in vitro* cell culture (Legant et al., Measurement of mechanical tractions exerted by cells in three-dimensional matrices, Nat Methods, 2010;7(12):969-71; Lesman et al., Cell tri-culture for cardiac vascularization, Methods Mol Biol, 2014;1181:131-7). However, the control population of embryos cultured in the presence of the composition of Group I and transferred to the collagen matrix failed to implant and the embryos do not progress. Since collagen polymerization occurs during the first hour after embryo embedding, these results discard the possibility that the process of deformation of the matrix is produced by heterogeneities in the polymerization of collagen when embryos are inserted. On the contrary, said deformations happen gradually over time, so their presence suggests the active mechanical action of the embryos. It is also surprising that supplementation by the composition of the present invention not only improves the implantation of the embryo but also extends its life until at least day E9.5.

## Claims

1. Composition for improving the implantation of mammalian embryos, comprising the supernatant of Fraction II+III and the Fraction V of the Cohn method wherein in said composition human serum albumin (HSA) is between 90% and 96% of the total proteins in the composition, alfa and beta globulins are between 3.5% and 9.99% of the total proteins in the composition, and gamma globulin is between 0.01% and 0.5% of the total proteins in the composition wherein said HSA is at a final concentration of 50 mg/ml to 250 mg/ml.

2. Composition according to claim 1, **characterized in that** the supernatant of Fraction II+III of the Cohn method is further processed by dilution, clarification, diafiltration, nanofiltration, concentration by ultrafiltration, sterilisation, filling into vials and final lyophilisation of said vials, prior to submission of said vials to gamma irradiation, or a combination thereof.

3. Composition according to claim 1, **characterized in that** the Fraction V of the Cohn method is further processed by diafiltration, heat treatment, sterilisation, filling into vials and final pasteurization of said vials, prior to submission of said vials to quarantine, or a combination thereof.

4. Composition according to claims 1 to 3, **characterized in that** the supernatant of Fraction II+III and the Fraction V, as such or as further processed production intermediates or final products, are submitted to one or more production steps with capacity to eliminate pathogens.

5. Composition according to any one of the preceding claims, **characterized in that** said HSA is at a final concentration of 100 mg/ml.

6. Method for preparation of a embryo culture medium comprising the composition according to any of the claims 1 to 5, comprising the following steps:
a) obtaining the composition according to claim 1;
b) adding 10-50% (v/v) of said composition to a mammalian embryo culture.

7. Method for preparation according to claim 6, **characterized in that** the composition is present in the embryo culture medium at 20% (v/v).

8. Use of the composition according to claims 1 to 5, for the culture of pre-implantation mammalian embryos.

## Patentansprüche

1. Zusammensetzung zur Verbesserung der Implantation von Säugetierembryonen, die den Überstand der Fraktion II+III und die Fraktion V der Cohn-Methode aufweist, wobei in der Zusammensetzung humanes Serumalbumin (HSA) zwischen 90% und 96% aller Proteine in der Zusammensetzung ausmachen, Alfa- und Betaglobuline zwischen 3,5% und 9,99% aller Proteine in der Zusammensetzung ausmachen und Gammaglobulin zwischen 0,01% und 0,5% aller Proteine in der Zusammensetzung ausmachen, und wobei das HSA eine endgültige Konzentration von 50 mg/ml bis 250 mg/ml aufweist.

2. Zusammensetzung nach Anspruch 1, **dadurch gekennzeichnet, dass** der Überstand der Fraktion II+III der Cohn-Methode weiterhin durch eine Verdünnung, Klärung, Diafiltration, Nanofiltration, Konzentration durch eine Ultrafiltration, Sterilisation, Abfüllung in Fläschchen und/oder finale Lyophilisation der Fläschchen vor der Aussetzung der Fläschchen an eine Gamma-Bestrahlung verarbeitet wird.

3. Zusammensetzung nach Anspruch 1, **dadurch gekennzeichnet, dass** die Fraktion V der Cohn-Methode weiterhin durch eine Diafiltration, Wärmebehandlung, Sterilisation, Abfüllung in Fläschchen und/oder endgültige Pasteurisierung der Fläschchen vor der Aussetzung der Fläschchen an eine Quarantäne verarbeitet wird.

4. Zusammensetzung nach einem der Ansprüche 1 bis 3, **dadurch gekennzeichnet, dass** der Überstand der Fraktion II+III und die Fraktion V so wie sie sind oder zu Zwischen- oder Endprodukten verarbeitet einem oder mehreren Produktionsschritten mit einer Kapazität zum Eliminieren von Pathogenen unterzogen werden.

5. Zusammensetzung nach einem der vorstehenden Ansprüche, **dadurch gekennzeichnet, dass** das HSA eine endgültige Konzentration von 100 mg/ml aufweist.

6. Verfahren zum Vorbereiten eines Embryonenkulturmediums, das die Zusammensetzung gemäß einem der Ansprüche 1 bis 5 umfasst, wobei das Verfahren die folgenden Schritte umfasst:
a) Erhalten der Zusammensetzung gemäß dem Anspruch 1,
b) Hinzufügen von 10-50% (v/v) der Zusammensetzung zu einer Säugetierembryonenkultur.

7. Verfahren zum Vorbereiten nach Anspruch 6, **dadurch gekennzeichnet, dass** die Zusammensetzung in dem Embryonenkulturmedium mit einem Anteil von 20% (v/v) vorgesehen ist.

8. Verwendung der Zusammensetzung gemäß den Ansprüchen 1 bis 5 für das Kultivieren von Präimplantation-Säugetierembryonen.

## Revendications

1. Composition pour améliorer l'implantation d'embryons de mammifères, comprenant le surnageant de la fraction II+III et la fraction V de la méthode Cohn, dans laquelle, dans ladite composition, l'albumine sérique humaine (HSA) représente entre 90 % et 96 % des protéines totales de la composition, les globulines alfa et bêta représentent entre 3.5 % et 9,99 % des protéines totales de la composition, et les gammaglobulines entre 0,01 % et 0,5 % des protéines totales de la composition dans laquelle ladite HSA est à une concentration finale de 50 mg/ml à 250 mg/ml.

2. Composition selon la revendication 1, **caractérisée par le fait que** le surnageant de la fraction II+III de la méthode de Cohn est en outre traité par dilution, clarification, diafiltration, nanofiltration, concentration par ultrafiltration, stérilisation, remplissage de flacons et lyophilisation finale desdits flacons, avant de soumettre lesdits flacons à une irradiation gamma, ou une combinaison de ceux-ci.

3. Composition selon la revendication 1, **caractérisée par le fait que** la fraction V de la méthode de Cohn est encore traitée par diafiltration, traitement thermique, stérilisation, remplissage de flacons et pasteurisation finale desdits flacons, avant la mise en quarantaine desdits flacons, ou une combinaison de ces procédés.

4. Composition selon les revendications 1 à 3, **caractérisée par le fait que** le surnageant de la fraction II+III et la fraction V, en tant que tels ou en outre en tant que produits de production traités intermédiaires ou finaux, sont soumis à une ou plusieurs étapes de production permettant d'éliminer les agents pathogènes.

5. Composition selon l'une quelconque des revendications précédentes, **caractérisée par le fait que** ladite HSA est à une concentration finale de 100 mg/ml.

6. Procédé de préparation d'un milieu de culture d'embryon comprenant la composition selon l'une quelconque des revendications 1 à 5, comprenant les étapes suivantes :
a) l'obtention de la composition selon la revendication 1 ;
b) l'ajout de 10 à 50 % (v/v) de ladite composition à une culture d'embryon de mammifère.

7. Procédé de préparation selon la revendication 6, **caractérisé par le fait que** la composition est présente dans le milieu de culture d'embryon à 20% (v/v).

8. Utilisation de la composition selon les revendications 1 à 5, pour la culture d'embryons préimplantatoires de mammifères.
